# EUROPÄISCHE PATENTSCHRIFT

(11) **EP 1 753 778 B1**
(45) Veröffentlichungstag und Bekanntmachung des Hinweises auf die Patenterteilung: **15.04.2009**
(21) Anmeldenummer: 05748375.2
(22) Anmeldetag: 07.06.2005
(51) Int. Cl.: C07K 14/505, C07K 1/16, C07K 1/18, C07K 1/20, C07K 1/22

(54) **VERFAHREN ZUR REINIGUNG VON ERYTHROPOIETIN**
METHOD FOR PURIFYING ERYTHROPOIETIN
PROCEDE POUR PURIFIER DE L'ERYTHROPOIETINE

(30) Priorität: 08.06.2004 DE 102004027816
(43) Veröffentlichungstag der Anmeldung: 21.02.2007
(73) Patentinhaber: Bioceuticals Arzneimittel AG, 61118 Bad Vilbel (DE)
(72) Erfinder: SCHUMANN, Christof, 35767 Breitscheid-Erdbach (DE); MACK, Michael, 61118 Bad Vilbel (DE); HESSE, Jan-Ole, 61231 Bad Nauheim (DE)
(74) Vertreter: Neuefeind, Regina
(86) Internationale Anmeldenummer: PCT/EP2005/006099
(87) Internationale Veröffentlichungsnummer: WO 2005/121173

(56) Entgegenhaltungen:
- EP-A- 1 428 878
- WO-A-00/27869
- WO-A-03/045996
- US-A1- 2002 146 771
- GOKANA A ET AL: "Chromatographic separation of recombinant human erythropoietin isoforms" JOURNAL OF CHROMATOGRAPHY A, ELSEVIER, AMSTERDAM, NL, Bd. 791, Nr. 1-2, 12. Dezember 1997 (1997-12-12), Seiten 109-118, XP004107601 ISSN: 0021-9673

## Beschreibung

Die vorliegende Erfindung betrifft ein Verfahren zur Herstellung von rekombinantem Erythropoietin, welches einen besonders hohen Reinheitsgrad (≥ 98 %) aufweist. Das Verfahren umfasst mindestens 5 chromatographische Reinigungsschritte, nämlich mindestens zwei Anionenaustausch-Chromatographien, mindestens eine hydrophobe Interaktionschromatographie, mindestens eine Affinitätschromatographie und mindestens eine Hydroxyapatit-Chromatographie. In bevorzugten Ausführungsformen verzichtet das Verfahren auf jegliche Ausschlusschromatographie und jegliche Reversed-Phase-Chromatographie. Insbesondere betrifft die Erfindung ein Verfahren, welches die folgenden chromatographischen Reinigungsschritte in der angegebenen Reihenfolge umfasst: i) eine erste Anionenaustausch-Chromatographie, ii) eine Affinitätschromatographie, bei der es sich vorzugsweise um eine Farbstoffaffinitätschromatographie handelt, iii) eine hydrophobe Interaktionschromatographie, eine iv) eine Hydroxyapatit-Chromatographie und v) eine zweite Anionenaustausch-Chromatographie.

Erythropoietin, kurz EPO genannt, ist ein Glykoprotein, das die Bildung von Erythrocyten im Knochenmark anregt. EPO wird hauptsächlich in den Nieren gebildet und gelangt von dort aus über den Blutkreislauf zu seinem Zielort. Bei Niereninsuffizienz produzieren die geschädigten Nieren zu wenig oder überhaupt kein EPO, was zur Folge hat, dass aus den Stammzellen des Knochenmarks zu wenig Erythrocyten hervorgehen. Diese renale Anämie kann durch Verabreichung von EPO in physiologischen Mengen, die die Bildung von Erythrocyten im Knochenmark stimulieren, behandelt werden. Das zur Verabreichung verwendete EPO kann entweder aus Humanurin gewonnen oder durch gentechnologische Methoden erzeugt werden. Da EPO im menschlichen Körper nur in geringen Spuren enthalten ist, ist die Isolierung von EPO aus der natürlichen Quelle für therapeutische Anwendungen praktisch unmöglich. Daher bieten gentechnische Methoden die einzige wirtschaftliche Möglichkeit, diesen Stoff in größeren Mengen zu produzieren.

Die rekombinante Herstellung von Erythropoietin ist seit der Identifizierung des humanen Erythropoietin-Gens im Jahr 1984 möglich. Seit Anfang der 90er Jahre sind verschiedene Arzneimittel entwickelt worden, die humanes Erythropoietin enthalten, das auf gentechnologischem Weg in eukaryontischen Zellen, vor allem in CHO-Zellen (Chinese Hamster Ovary) produziert wurde. Die Herstellung von rekombinantem humanen Erythropoietin ist beispielsweise beschrieben in EP-A-0 148 605 und EP-A-205 564.

Die rekombinante Herstellung von Erythropoietin erfolgt üblicherweise in CHO-Wirtszellen. Während diese früher in Kulturmedium kultiviert wurden, dem fötales Kälberserum und manchmal auch Rinderinsulin zugesetzt war, findet die Kultivierung heutzutage regelmäßig in serum- und proteinfreiem Medium statt. Auf diese Weise wird das Risiko von Kontaminationen mit bovinen Proteinen, bovinen Viren, boviner DNA oder anderen unerwünschten Substanzen, die aus den früher eingesetzten Zusätzen stammen, bereits durch die Kultivierung reduziert werden. Für die Kultivierung von eukaryontischen Zellen geeignete, serum- und proteinfreie Medien werden von verschiedenen Anbietern angeboten, z.B. das Medium MAM-PF2, vertrieben von u.a. Bioconcept, Allschwil, Schweiz, oder die Medien DMEM und DMEM/F1.2 angeboten z.B. von Invitrogen/Gibco, Eggenstein, Deutschland.

Auch verschiedene chromatographische Reinigungsverfahren für Erythropoietin sind bereits im Stand der Technik beschrieben worden. EP-A-0 228 452 beschreibt ein Verfahren zur Aufreinigung von biologisch aktivem Erythropoietin aus einer Flüssigkeit, umfassend die chromatographischen Schritte Anionenaustausch-Chromatographie und Reversed-Phase-Chromatographie.

In EP-A-0 267 678 wird die Aufreinigung eines in-serumfreier Kultur hergestellten Erythropoietins beschrieben, wobei aufeinander folgend eine Dialyse, eine Ionenaustausch-Chromatographie, eine präparative Reversed-Phase-HPLC und eine Gelfiltrationschromatographie durchgeführt werden. Dabei kann der Gelfiltrationschromatographieschritt durch eine Ionenaustauschchromatographie ersetzt werden. Ebenso wird vorgeschlagen, vor der (ersten) Ionenaustauschchromatographie eine Farbstoffaffinitätschromatographie an einer Blue Trisacryl-Säule durchzuführen.

In EP-A-0 830 376 ist ein Verfahren zur Reinigung von Erythropoietin beschrieben, bei dem EPO aus dem Kulturüberstand im ersten Schritt der chromatographischen Aufreinigung einer Farbstoffaffinitätschromatographie unterzogen wird. Im zweiten Schritt folgt eine Chromatographie an einem hydrophobisierten Träger, gefolgt von einer Hydroxyapatit-Chromatographie. Hieran schließt sich dann eine Reversed-Phase-HPLC an, gefolgt von einer Anionenaustausch-Chromatographie als letztem Chromatographieschritt.

EP-A-1 127 063 beschreibt ein Aufreinigungsverfahren für Erythropoietin, das folgende Schritte umfasst: differentielle Präzipitation, hydrophobe Interaktionschromatographie, Diafiltration, Anionenaustausch-Chromatographie, Kationenaustausch-Chromatographie und Size-Exclusion-Chromatographie. Die einzelnen Reinigungsschritte werden in EP-A-1 127 063 in der genannten Reihenfolge durchgeführt. In einer Variante des Verfahrens umfasst die Reinigung die folgenden Schritte: differentielle Präzipitation, hydrophobe Interaktionschromatographie, Diafiltration, Anionenaustausch-Chromatographie, Kationenaustausch-Chromatographie, eine weitere Diafiltration und Size-Exclusion-Chromatographie. In jedem Fall sieht das Verfahren im ersten Schritt eine Präzipitation, gefolgt von einer Zentrifugation vor. Ebenso ist eine Gelfiltration zum Abschluss der chromatographischen Reinigung zwingend vorgesehen.

Die internationale Anmeldung WO-A-03/045996 beschreibt ein Reinigungsverfahren für EPO, umfassend eine Anionenaustausch-Chromatographie, gefolgt von einer Reversed-Phase-Chromatographie und einer weiteren Anionenaustausch-Chromatographie. An die zweite Anionenaustausch-Chromatographie schließt sich eine Size-Exclusion-Chromatographie unter Einsatz eines Gelfiltrationsmediums an.

Die Reinigung von Erythropoietin ist auch Gegenstand von EP-A-0 428 267. Hier wird eine Chromatographie an einer Q Sepharose-Säule durchgeführt, teilweise gefolgt von Reversed-Phase-Chromatographie und Gelfiltration.

Aufgabe der vorliegenden Erfindung ist es, ein Aufreinigungsverfahren für Erythropoietin aufzuzeigen, das auf kostenintensive Chromatographieschritte möglichst ebenso verzichtet wie auf aufwendige Schritte, die zudem den Einsatz unerwünschter Reagenzien erforderlich machen können. Das mit dem erfindungsgemäßen Reinigungsverfahren erhaltene Erythropoietin soll den von den Zulassungsbehörden bzw. den in der Europäischen Pharmacopoeia definierten Reinheitskriterien entsprechen. Insbesondere soll der Gehalt von Proteinen, die aus der Wirtszelle stammen (Wirtszellprotein), unter 100 ppm liegen. Auch soll der Gehalt an DNA aus der Wirtszelle unter 100 pg/mg Erythropoietin liegen. Schließlich soll das durch die Reinigung erhaltene Erythropoietin hinsichtlich seiner Isoformen-Zusammensetzung dem in der Europäischen Pharmacopoeia (Ph. Eur.; 01/2002:1316) definierten Standard entsprechen.

Auch soll das Verfahren möglichst ohne eine Reversed-Phase-Chromatographie, wie z.B. eine RP-HPLC auskommen. Bei dieser Art von Chromatographie werden üblicherweise Reagenzien wie Acetonitril eingesetzt, die anschließend schwierig von dem Protein zu entfernen sind und für Menschen schädlich sein könnten. Ein anderer Nachteil der RP-HPLC ist, dass häufig kostenintensive organische Lösungsmittel eingesetzt werden, die die Reinigungskosten erhöhen, außerdem sind organische Lösungsmittel im Hinblick auf Umweltschäden bedenklich und auch in der Handhabung schwierig und gefährlich. Insgesamt sind die bei der Reversed-Phase-Chromatographie verwendeten Mittel häufig unerwünscht.

Es ist ersichtlich, dass an das aus der Reinigung erhaltene Erythropoietin-Produkt hohe Maßstäbe gerichtet werden hinsichtlich Reinheit und Glykosylierungsmuster. Diese hohen Anforderungen können nur durch ein speziell auf Erythropoietin ausgerichtetes Reinigungsverfahren erfüllt werden, das das Ergebnis umfangreicher Studien und Analysen ist.

Diese und weitere Aufgaben werden durch das in Anspruch 1 angegebene Reinigungsverfahren gelöst. Bevorzugte Ausführungsformen sind in den abhängigen Patentansprüchen beschrieben.

Die Erfindung betrifft somit ein Verfahren zur Aufreinigung von Erythropoietin aus einer Lösung, insbesondere einem Kulturüberstand, wobei die folgenden Schritte a) bis c) in der angegebenen Reihenfolge durchgeführt werden: im Schritt a) eine erste Anionenaustausch-Chromatographie, im Schritt b) eine Affinitätschromatographie, eine hydrophobe Interaktionschromatographie und eine Hydroxyapatitchromatographie, in der angegebenen Reinenfolge und im Schritt c) eine zweite Anionenaustausch-Chromatographie.

Das erfindungsgemäße Reinigungsverfahren macht somit von mindestens vier verschiedenen chromatographischen Trennmethoden Gebrauch, nämlich (i) der des Ionenaustausches auf der Grundlage der kompetitiven Wechselwirkung geladener Ionen, (ii) der hydrophoben Interaktion, die sich dadurch auszeichnet, dass die unpolaren Oberflächenregionen eines Proteins bei hohen Salzkonzentrationen an die schwach hydrophoben Liganden einer stationären Phase adsorbieren, (iii) der Affinität, die auf der spezifischen und reversiblen Adsorption eines Moleküls an einen individuellen, matrixgebundenen Bindungspartner beruht, und (iv) der Hydroxyapatit-Chromatographie, die auf dem Einsatz von anorganischen Hydroxyapatitkristallen basiert.

Diese genannten Chromatographieprinzipien werden auch in Fachkreisen entsprechend unterschieden (siehe z.B. Bioanalytik, F. Lottspeich, H. Zorbas (Hrsg.), Heidelberg, Berlin, Spektrum Akad. Verlag 1998). Trotzdem sei betont, dass es sich bei der in Schritt b) zwingend vorgesehenen Affmitätschromatographie nicht um eine Hydroxyapatit-Chromatographie handelt. Vielmehr umfasst Schritt b) sowohl eine Affinitätschromatographie als auch eine Hydroxyapatit-Chromatographie.

In einer bevorzugten Ausführungsform umfasst das EPO-Reinigungsverfahren eine erste Anionenaustausch-Chromatographie, eine Affinitätschromatographie, eine hydrophobe Interaktionschromatographie, eine Hydroxyapatitchromatographie und eine zweite Anionenaustausch-Chromatographie, in der genannten Reihenfolge.

Bei der Affinitätschromatographie handelt es sich bevorzugt um eine Farbstoffaffinitätschromatographie.

Die zweite Anionenaustausch-Chromatographie erfolgt in einer bevorzugten Ausführungsform unter Durchführung eines sauren Waschschritts, mit dem die basischen Isoformen des Erythropoietins durch eine deutliche pH-Absenkung eluiert und somit vom Endprodukt abgetrennt werden. "Saurer Waschschritt" bedeutet dabei, dass der pH-Wert des Waschpuffers deutlich im saueren Bereich, vorzugsweise zwischen 2,0 und 5,5, besonders bevorzugt zwischen 3,0 und 4,5 und am meisten bevorzugt bei etwa 4,0 liegt. Als Puffer eignet sich vor allem ein Natriumacetat-Puffer. Dieser Chromatographieschritt ist demnach besonders wichtig im Hinblick auf das Glykosylierungsmuster des EPO-Endprodukts.

Die Kultivierung der Erythropoietin-produzierenden Wirtszellen erfolgt in einem Kulturmedium, das proteinfrei und frei von tierischen Komponenten ist.

In bevorzugten Ausführungsformen findet in keinem Stadium der EPO-Aufreinigung eine Reversed-Phase-Chromatographie statt. Auch eine Gelfiltration wird möglichst vermieden.

Es wurde gefunden, dass das mit dem erfindungsgemäßen Verfahren erhaltene Erythropoietin einen Wirtszellproteingehalt von < 100 ppm und einen Wirtszell-DNA-Gehalt von < 100 pg/mg aufweist. Besonders die unzureichende Abreicherung von Wirtszellprotein ist ein häufiges Problem bei Reinigungsverfahren des Standes der Technik. Das erfindungsgemäße Verfahren bietet hier besondere Vorteile, da eine zuverlässige Abreicherung unter 100 ppm erreicht wird.

Das mit dem erfmdungsgemäßen Verfahren erhaltene Erythropoietin hat eine Reinheit von mindestens 95%, vorzugsweise mindestens 98% und besonders bevorzugt von mindestens 99%, wobei die Reinheit mittels analytischer RP-HPLC bestimmt wird. Die hierzu durchgeführte RP-HPLC dient aber nur Analysezwecken, im Rahmen der Aufreinigung wird möglichst keine RP-HPLC durchgeführt.

Die Aktivität des Proteins sollte mindestens 100.000 IE/mg betragen, vorzugsweise mindestens 110.000 IE/mg und besonders bevorzugt mindestens 120.000 IE/mg (siehe auch Europäische Pharmacopoeia 01/2002:1316).

Die Erfindung beschreibt auch pharmazeutische Zubereitungen, die das erfindungsgemäß aufgereinigte Erythropoietin enthalten. EPO wird üblicherweise in flüssiger Form formuliert und wird als solche intravenös oder subkutan injiziert. Geeignete Hilfsstoffe in Flüssigformulierungen von EPO sind z.B. Puffer wie z.B. Phosphatpuffer, Salze wie beispielsweise Natriumchlorid, Stabilisatoren für EPO wie z.B. Aminosäuren, Zucker und Zuckeralkohole, sowie Tenside, wie z.B. Polysorbat 20/80. Beispiele für Formulierungen sind beschrieben in EP-A-0 306 824, EP-A-0 607 156 und EP-A-0 909 564, siehe auch Handelsprodukte NeoRecormon^{®}, Erypo^{®} in ROTE LISTE 2004.

Das erfindungsgemäß aufgereinigte Erythropoietin ist bevorzugt rekombinantes humanes Erythropoietin, hergestellt in eukaryontischen Zellen. Bevorzugt wird das rekombinante Erythropoietin in Säugerzellen, besonders bevorzugt in CHO-Zellen hergestellt, wie z.B. beschrieben in EP-A-0 205 564 und EP-A-0 148 605. Die Fermentation erfolgt nach herkömmlichen Protokollen in kommerziell erhältlichen Kulturmedien.

Unter "Erythropoietin" wird im Rahmen der vorliegenden Erfindung jedes Protein verstanden, das in der Lage ist, die Erythrocyten-Bildung im Knochenmark zu stimulieren und gemäß dem in der Europäischen Pharmacopoeia (Ph. Eur.; 01/2002:1316) beschriebenen Assay eindeutig als Erythropoietin identifiziert werden kann (Bestimmung der Aktivität in polycythämischen oder normocythämischen Mäusen). Bei dem Erythropoietin kann es sich um das wildtypische humane Eythropoietin oder um eine Variante davon mit einem oder mehreren Aminosäureaustauschen, -deletionen oder -additionen handeln. Ebenso kann es sich bei dem in der erfindungsgemäßen Formulierung enthaltenen Erythropoietin um ein Konjugat handeln, in dem das Protein z.B. in konjugierter Form mit Polymeren, wie z.B. Polyalkylenglykolen vorliegt, sog. PEGyliertes Erythropoietin.

Unter "Reinigung von Erythropoietin" oder "Anreicherung von Erythropoietin" wird vorliegend verstanden, dass das Protein Erythropoietin aus einem Gemisch in sehr reiner Form erhalten wird, das in dem Gemisch enthaltene Erythropoietin wird also angereichert, bis im Wesentlichen neben Erythropoietin keine weiteren Proteine mehr enthalten sind.

Die in dem erfindungsgemäßen Verfahren genutzten chromatographischen Prinzipien sind dem Fachmann geläufig, jedenfalls in gängigen Manuals oder Protokollen der Anbieter von Chromatographie-Matrices ausführlich beschrieben. Geeignete Matrices und Hintergrundinformationen sowie Anleitungen zur Durchführung der verschiedenen Chromatographien finden sich z.B. im Produk-tkatalog und den Produktinformationen von Amersham Biosciences (siehe auch www.amershambiosciences.com) oder auch im Produktkatalog von Bio-Rad (siehe auch www.bio-rad.com).

Die Anionenaustausch-Chromatographien können mit herkömmlichen, kommerziell erhältlichen Anionaustausch-Harzen oder Membranen durchgeführt werden. Typische Anionenaustausch-Harze, die eingesetzt werden können, umfassen funktionelle Gruppen wie Diethylaminoethyl (DEAE), hier sind zu nennen DEAE-Sepharose (Amersham Biosciences), Macroprep DEAE (Bio-Rad), Fractorgel EMD DEAE (Merck); quartäres Aminoethyl (QAE), hier ist zu nennen, Toyopearl QAE (Toyo Biosep); quartäres Ammonium, hier sind zu nennen Q-Sepharose XL (Amersham Biosciences), Q-Sepharose FF (Amersham Biosciences), Resource Q (Amersham Biosciences), Source 30 Q (Amersham Biosciences), Macro Prep High Q (Bio-Rad), Toyopearl Super Q (Toyo Biosep); Dimethylaminoethyl (DMAE), hier ist zu nennen Fractogel EMD DMAE (Merck); Trimethylaminoethyl (TMAE), hier ist zu nennen Fractogel EMD TMAE (Merck); Sartobind Membraneadsorber (MA) Q100 (Sartorius).

Bevorzugte Anionenaustauscher sind Harze mit quartären oder tertiären Ammoniumliganden. So wird z.B. in einer bevorzugten Ausführungsform des erfindungsgemäßen Verfahrens in der ersten und zweiten Anionenaustausch-Chromatographie jeweils Q Sepharose XL oder Source 30 Q (beide erhältlich von Amersham Biosciences) eingesetzt. Besonders bevorzugt wird in der ersten Anionenaustausch-Chromatographie Q Sepharose XL und in der zweiten Anionenaustausch-Chromatographie Source 30 Q verwendet.

Die Affinitätschromatographie kann ebenfalls mit herkömmlichen, kommerziell erhältlichen Harzen durchgeführt werden. Zu nennen sind hier beispielhaft Dye-Sepharose, Heparin-Sepharose, HiTrap Blue HP-Säulen (Cibacron Blue F3G-A), Blue Sepharose (Cibacron Blue F3G-A), Peptid-Ligand-Affinitätsharze, Antikörper-Affinitätsharze, Lectin-Affinitätsharze, Affinitätschromatographie an immobilisierter DNA bzw. immobilisierten Nukleotiden und an gruppenspezifischen Adsorptionsmitteln wie z.B. an Agarose-gekoppelter Gelantine.

Bevorzugt handelt es sich um eine Farbstoffaffinitätschromatographie, insbesondere unter Einsatz von Blue Sepharose (z.B. Blue Sepharose 6 Fast Flow von Amersham Biosciences). Aber auch andere Farbstoffaffinitätsmatrices sind geeignet, wie z.B. das Produkt DyeMatrex von der Firma Millipore.

Auch die hydrophobe Interaktionschromatographie kann mit üblichen Matrices durchgeführt werden. Geeignet sind Matrices wie Butyl-, Phenyl-, Propyl- oder Octyl-Sepharose (Amersham Biosciences), Macro-Prep Methyl oder t-Butyl (Bio-Rad) und Fractogel EMD mit Propyl- oder Phenyl-Liganden (Merck). Bevorzugt handelt es sich um Butyl Sepharose (z.B. Butyl Sepharose 4 Fast Flow von Amersham Biosciences).

Für die Hydroxyapatit-Chromatographie können übliche Hydroxyapaptit-Materialien eingesetzt werden. Bei Hydroxyapatit handelt es sich um eine Form von Calciumphosphat. Bevorzugt wird CHT keramisches Hydroxyapatit (Bio-Rad) eingesetzt, besonders bevorzugt CHT keramisches Hydroxyapatit Typ I (Bio-Rad).

Das Isoformenmuster des EPO-Endprodukts, also erhalten mittels des erfmdungsgemäßen Verfahrens und bestimmt nach der zweiten Anionenaustausch-Chromatographie, ist vergleichbar mit dem BRP-EPO-Standard (siehe Europäische Pharmacopoeia 01/2002:1316).

Die folgenden Beispiele sollen die Erfindung erläutern, ohne sie einzuschränken.

### Beispiele:

EPO wird in CHO-Zellen hergestellt. Die Fermentation erfolgt nach Standardprotokollen, wie sie in der Patent- und wissenschaftlichen Literatur für eukaryontische, insbesondere CHO-Zellen beschrieben sind. Die Kultivierung erfolgt in Kulturmedium, das proteinfrei und frei von tierischen Komponenten ist (z.B. MAM-PF2, erhältlich von Bioconcept, Allschwil, Schweiz, nach den Empfehlungen des Anbieters). Die Ernte findet nach einer maximal sieben Tage dauernden Produktionsphase statt. Dabei werden die Zellen durch einen Tiefenfilter und anschließende 0,2 µm-Filtration abgetrennt. Die Zellen können alternativ durch Abzentrifugieren entfernt werden. Das zellfreie Filtrat wird anschließend mittels Ultrafiltration um ca. den Faktor 10 aufkonzentriert und gegen Phosphatpuffer diafiltriert. Die Diafiltration dient zur Reduzierung der Leitfähigkeit unter 5 mS/cm, um die Proteinlösung für den ersten chromatographischen Schritt, den sog. Capture-Schritt, vorzubereiten.

### Übersicht Aufreinigungsverfahren

### 1. Chromatographie-Schritt (Capture, IEX 1)

Als "Capture"-Schritt wird eine Ionenaustauschchromatographie (IEX) an Q Sepharose XL durchgeführt. In diesem ersten Reinigungsschritt wird der Wirkstoff Erythropoietin aufkonzentriert. Außerdem dient dieser Schritt der Überführung des Wirkstoffs in eine stabilere Lagerform.

Das Waschen erfolgt mit 20 mM Na-Phosphat, pH 7,5, die Elution mit 0,3 M Natriumchlorid bei pH 7,5. Im Anschluss an die Anionenaustausch-Chromatographie kann eine 0,2 µm-Filtration durchgeführt werden.

### 2. Chromatographie-Schritt (Affinität)

Im nächsten Schritt erfolgt eine Affinitätschromatographie an Blue Sepharose 6 FF (bezogen von Amersham Biosciences). Nach Austausch des Puffers auf der Säule und einem zusätzlichen Waschschritt erfolgt die Elution in 1 M Natriumchlorid. Das Eluat wird für die anschließende hydrophobe Interaktionschromatographie mit salzhaltigem Puffer und Isopropanol versetzt.

### 3. Chromatographie-Schritt (HIC)

Das mit salzhaltigem Puffer und Isopropanol versetzte Eluat wird im nächsten Schritt auf eine hydrophobe Interaktionschromatographie-Säule (HIC; Butyl Sepharose 4 FF, bezogen von Amersham Biosciences) aufgetragen. Nach einem Waschschritt (2 M Natriumchlorid in 10% Isopropanol) erfolgt die Elution des Wirkstoffs mit 0,75 M Natriumchlorid in 23% Isopropanol (v/v).

### 4. Chromatographie-Schritt (Hydroxyapatit)

Das Eluat aus der hydrophoben Interaktionschromatographie wird anschließend einer Hydroxyapatit-Chromatographie (CHT-I Ceramic Hydroxyapatite, bezogen von Bio-Rad) unterzogen. Vorher kann es verdünnt werden. Vorzugsweise erfolgt das Verdünnen, indem das Eluat der HIC-Säule direkt in Tris-Puffer (20 mM Tris/HCl, 5 mM CaCl₂, pH 6,9) eingebracht wird. Die finale Isopropanolkonzentration beträgt nach der Verdünnung vorzugsweise etwa 9%. Diese Lösung wird dann direkt auf die Hydroxyapatit-Säule aufgetragen.

Nach Waschen mit 10 mM Kaliumphosphat-Puffer erfolgt die Elution des Erythropoietins. Der pH-Wert des Eluats wird mit HCl auf pH 7,4 eingestellt.

### 5. Chromatographie-Schritt (IEX 2)

Für den nachfolgenden und vorzugsweise letzten chromatographischen Schritt wird die EPO-haltige Lösung auf eine Ionenaustausch-Matrix (Source 30 Q, bezogen von Amersham Biosciences) geladen. Dieser Chromatographieschritt beinhaltet einen sauren Waschschritt mit Natriumacetat (pH 4,0), um basische Isoformen des Wirkstoffs abzureichern. Nach Einstellen des pH-Wertes durch einen weiteren Waschschritt auf pH 7,4 erfolgt die Elution des Erythropoietins mit 200 mM Natriumchlorid.

Anschließend kann wiederum eine 0,2 µm-Filtration im Rahmen der Virusfiltration durchgeführt werden. Die Wirkstofflösung wird als Bulk bezeichnet und kann mittels flüssigem Stickstoff schockgefroren und dann bei -80°C eingelagert werden.

Das erhaltene Erythropoietin kann in Form einer Flüssigformulierung zusammen mit üblichen pharmazeutisch annehmbaren Hilfsstoffen formuliert werden.

### Die einzelnen Chromatographie-Schritte im Detail

### 1. Erste Anionenaustausch-Chromatographie (Capture-Schritt)

Die Equilibrierung der Q Sepharose XL-Matrix (0,6 L ± 0,05 L) erfolgt mit 20 mM Na-Phosphat, pH 7,5 (bis hinter der Säule der pH-Wert 7,5 ± 0,3 und die Leitfähigkeit < 5 mS/cm beträgt). Dann erfolgt der Probenauftrag. Anschließend wird die Säule mit dem Equilibrierungspuffer, also 20 mM Na-Phosphat, pH 7,5, gewaschen. Für die anschließende Elution wird 20 mM Na-Phosphat, 300 mM NaCl, pH 7,5 verwendet.

Auf einen sauren Waschschritt wird im Rahmen des Capture-Schritts in der Regel verzichtet.

Die Reinheit des eluierten Erythropoietins beträgt > 65 %, gemessen mit RP-HPLC.

Im Anschluss an den Capture-Schritt kann eine 0,2 µm-Filtration erfolgen. Bevorzugt wird das Eluat direkt während der Elution mit Natriumchlorid durch einen 0,2 µm-Filter gepumpt.

### 2. Blue Sepharose-Affinitätschromatographie

Die Equilibrierung der Säule erfolgt mit 20 mM Na-Phosphat, 0,1 M NaCl, pH 7,5. Anschließend wird die aus dem Capture-Schritt erhaltene Probe auf die Säule aufgetragen, wobei diese vorher zur Vorbereitung auf die Affinitätschromatographie mit 20 mM Na-Phosphat, pH 7,5 verdünnt werden kann.

Anschließend wird einmal gewaschen mit 20 mM Tris-HCl, 0,1 M NaCl, pH 7,5. Der zweite Waschschritt erfolgt mit 20 mM Tris/HCl, 5 mM Nach, 0,1 M NaCl, pH 7,5. Die Elution erfolgt mit 100 mM Tris/HCl, 5 mM CaCl₂, 1 M NaCl, pH 7,5.

Die Blue Sepharose-Affinitätschromatographie dient u.a. der Abreicherung von Wirtszellprotein. Der Gehalt an Wirtszellprotein wird üblicherweise mittels ELISA bestimmt. Diese und andere Strategien zur Wirtszellprotein-Analyse können z.B. in Hoffman K. (2000) Biopharm, Vol. 13, Nr. 6, pp 38-45 nachgelesen werden.

Die Ausbeute von Erythropoietin, gemessen mit RP-HPLC, beträgt nach der Blue Sepharose-Affinitätschromatographie mindestens 65%, bevorzugt mindestens 70%. Die Reinheit, ebenfalls mit RP-HPLC gemessen, beträgt mindestens 90%, vorzugsweise mindestens 95%.

### 3. Hydrophobe Interaktionschromatographie (HIC)

Die hydrophobe Interaktionschromatographie wird mit Butyl Sepharose 4 FF als Matrix durchgeführt. Diese Matrix ist physikalisch stabil und erlaubt hohe Fließgeschwindigkeiten.

Die zur Bindung von EPO nötige Salzkonzentration (2 M NaCl) wird durch das Mischen des Eluats der Blue Sepharose 6 FF mit 4 M NaCl-haltigem Puffer eingestellt. Zusätzlich wird die Probe auf 10% Isopropanol (v/v) eingestellt.

Die Leitfähigkeit der für die Beladung der HIC-Säule vorgesehenen Probe sollte nach Möglichkeit zwischen 95 und 110 mS/cm liegen. Die Leitfähigkeit des Equilibrierungspuffer sollte ebenfalls in diesem Bereich liegen.

Die HIC-Säule wird mit 20 mM Tris/HCl, 2 M NaCl, 10% Isopropanol, pH 7,5 equilibriert. Nach dem Probenauftrag erfolgt das Waschen mit dem Equilibrierungspuffer. Die Elution erfolgt mit folgendem Elutionspuffer: 20 mM Tris/HCl, 5 mM CaCl₂, 0,75 M NaCl, 23% (v/v) Isopropanol, pH 6,9. Das Elutionsvolumen beträgt 1 Säulenvolumen.

Es hat sich gezeigt, dass im Rahmen des hier im Detail dargestellten Aufreinigungsprozesses eine Isopropanol-Konzentration von 23 % (v/v) im Elutionspuffer zu einer optimalen EPO-Elution bei gleichzeitig minimierten Verlusten führt.

Um eine Präzipitieren des EPO durch langes Einwirken von Isopropanol (23 %, v/v) zu vermeiden, wird während der Elution das Eluat direkt in Tris-Puffer eingebracht. Die Isopropanolkonzentration des verdünnten Eluats beträgt schließlich 9 % (v/v).

Die Ausbeute an Erythropoietin nach diesem Chromatographieschritt beträgt mindestens 65%, bevorzugt mindestens 70%, besonders bevorzugt mindestens 80%. Die Reinheit beträgt mindestens 92%, bevorzugt mindestens 95%.

### 4. Hydroxyapatit-Chromatographie

Das Eluat der Butyl Sepharose 4 FF-Chromatographie kann ohne weitere Konditionierung auf die Hydroxyapatit-Säule (HAP) geladen werden. Aus den Elutionsbedingungen der hydrophoben Interaktionschromatographie ergibt sich dadurch für das Equilibrieren der Säule ein Isopropanol-haltiger Tris-Equilibrierungspuffer. Nach dem Beladen der Säule wird das Isopropanol durch einen Waschschritt entfernt.

Die für den Hydroxyapatit-Chromatographieschritt eingesetzten Puffer sind wie folgt:
Equilibrierungspuffer: 20 mM Tris/HCl, 5 mM NaCl₂, 0,25 M NaCl, 9% Isopropanol, pH 6,9.

Der erste Waschschritt erfolgt mit dem Equilibrierungspuffer, der zweite Waschschritt mit: 10 mM Tris/HCl, 5 mM CaCl₂, pH 6,8. Der Elutionspuffer ist wie folgt:
10 mM Tris/HCl, 0,5 mM CaCl₂, 10 mM K₂HPO₄, pH 6,8.

Die Ausbeute des Hydroxyapatit-Chromatographieschritts beträgt mindestens 65%, vorzugsweise mindestens 70%. Die Reinheit des erhaltenen EPO beträgt mindestens 97%, vorzugsweise mindestens 98%.

Insgesamt dient die Chromatographie mittels Hydroxyapatit zur Entfernung des Isopropanols aus dem hydrophoben Interaktionschromatographieschritt. Die Elution des EPO von der Hydroxyapaptit-Säule kann vorzugsweise durch eine Stufenelution (0 auf 10 mM Kaliumphosphat) erfolgen. Aber auch ein linearer Gradient (bspw. von 0 auf 40 mM Kaliumphosphat) kann eingesetzt werden.

Im Anschluss an die Hydroxyapatitchromatographie wird in einer bevorzugten Ausführungsform des erfindungsgemäßen Verfahrens eine Virusfiltration durchgeführt. Diese Virusfiltration kann beispielsweise mit einem Planova 15N-Filter der Asahi Kasei-Gruppe durchgeführt werden. Die Filtermembran besitzt eine Porengröße von 15 nm und stellt auch die Abreicherung von Polioviren und Parvoviren sicher.

### 5. Zweite Ionenaustauschchromatographie

Als letzter Aufreinigungsschritt wird eine Ionenaustauschchromatographie mittels Source 30 Q durchgeführt. Der Schritt ist vor allem durch einen sauren Waschschritt gekennzeichnet, mit dem die basischen Isoformen des Erythropoietins durch eine pH-Absenkung eluiert und somit vom Endprodukt abgetrennt werden. Dieser Chromatographieschritt ist demnach besonders wichtig im Hinblick auf das Glykosylierungsmuster des EPO-Endprodukts.

Als Puffersystem für den zweiten Ionenaustauschschritt zur Reinigung von Erythropoietin wird ein Phosphatpuffer verwendet. Nach dem Laden der Probe und einem ersten Waschschritt wird ein "saurer" Waschschritt durchgeführt. Unter diesen Bedingungen eluieren basische Isoformen des Erythropoietins.

Die Equilibrierung und der erste Waschschritt erfolgen mit 10 mM Na-Phosphat, pH 7,4. Der saure Waschschritt erfolgt mit einem Waschpuffer mit einem Natriumacetat-Gehalt von 20 mM und einem pH-Wert von 4,0. Das Waschvolumen des sauren Waschschrittes beträgt 2 Säulenvolumen.

Anschließend wird in der Regel noch einmal mit einem Phosphatpuffer gewaschen, wodurch der pH-Wert auf 7,4 erhöht wird. Die Elution erfolgt mit einem Stufengradienten von 0 auf 0,2 M NaCl. Aber auch ein linearer Gradient, z.B. von 0 auf 0,5 M NaCl ist geeignet.

Der Elutionspuffer hat die Zusammensetzung: 20 mM Na-Phosphat, 0,2 M NaCl, pH 7,4. Die Elution erfolgt über 1,5 Säulenvolumen.

Die Ausbeute beträgt mindestens 65%, vorzugsweise mindestens 70% und besonders bevorzugt mindestens 75%. Die Reinheit beträgt mindestens 98%, vorzugsweise mindestens 99%.

Das schließlich erhaltene EPO-Produkt hat eine biologische Aktivität von ≥ 100.000 IU/mg im Bio-Assay, einen DNA-Gehalt von < 100 pg/mg Protein, eine EPO-Reinheit von ≥ 98 %, einen Wirtszellgehalt (HCP, host cell protein) von < 100 ppm und ein Isoformenmuster (in der CZE), das die Anforderungen der Europäischen Pharmacopoeia erfüllt.

Sämtliche Säulenchromatographien werden im Übrigen nach den Empfehlungen und Protokollen der Anbieter der Matrices bzw. der Säulen durchgeführt (z.B. bezüglich der Fließgeschwindigkeiten, der zum Waschen bzw, für die Elution eingesetzten Säulenvolumina, der Durchmesser und Betthöhen der Säulen etc.).

## Patentansprüche

1. Verfahren zur Anreicherung von Erythropoietin, hergestellt durch Kultivierung von eukaryontischen Wirtszellen in einem Kulturmedium, in einem Proteingemisch, wobei die folgenden Schritte a)-c) in der angegebenen Reihenfolge durchgeführt werden:
a) eine erste Anionenaustauschchromatographie,
b) eine Affinitätschromatographie, eine hydrophobe Interaktionschromatographie und eine Hydroxyapatitchromatographie in der angegebenen Reihenfolge, und
c) eine zweite Anionenaustauschchromatographie.

2. Verfahren nach Anspruch 1, wobei die Affinitätschromatographie als Farbstoffaffinitätschromatographie ausgestaltet ist.

3. Verfahren nach einem der vorangehenden Ansprüche, wobei die zweite Anionenaustauschchromatographie einen sauren Waschschritt umfasst.

4. Verfahren nach einem der vorangehenden Ansprüche, weiter umfassend mindestens einen Filtrationsschritt.

5. Verfahren nach Anspruch 4, wobei eine Filtration nach dem letzten chromatographischen Reinigungsschritt von Schritt b) stattfindet.

6. Verfahren nach Anspruch 4 oder 5, wobei eine Filtration nach der zweiten Anionenaustauschchromatographie stattfindet.

7. Verfahren nach einem der vorangehenden Ansprüche, wobei das Proteingemisch ein wirtszellfreies Filtrat des Kulturmediums ist, das vor der ersten Anionenaustauschchromatographie einer Diafiltration unterzogen wird.

8. Verfahren nach Anspruch 7, wobei das wirtszellfreie Filtrat vor der Diafiltration einer Ultrafiltration unterzogen wird.

9. Verfahren nach einem der vorangehenden Ansprüche, wobei das Kulturmedium proteinfrei und frei von tierischen Komponenten ist.

10. Verfahren nach einem der vorangehenden Ansprüche, wobei das Verfahren keine Reversed-Phase-Chromatographie umfasst.

11. Verfahren nach einem der vorangehenden Ansprüche, wobei das Verfahren keine Gelfiltration umfasst.

12. Verfahren nach einem der vorangehenden Ansprüche, wobei das Verfahren keine Proteinpräzipitation umfasst.

13. Verfahren nach einem der vorangehenden Ansprüche, wobei das Verfahren darüber hinaus keine weiteren chromatographischen Reinigungsschritte umfasst.

14. Verfahren nach einem der vorangehenden Ansprüche, wobei das angereicherte Erythropoietin nach der zweiten Anionenaustauschchromatographie einen Wirtszellproteingehalt von < 100 ppm aufweist.

15. Verfahren nach einem der vorangehenden Ansprüche, wobei das angereicherte Erythropoietin nach der zweiten Anionenaustauschchromatographie einen Wirtszell-DNA-Gehalt von < 100 pg/mg aufweist.

16. Verfahren nach einem der vorangehenden Ansprüche, wobei das angereicherte Erythropoietin nach der zweiten Anionenaustauschchromatographie eine Reinheit von ≥98 %, bestimmt mittels RP-HPLC, aufweist.

17. Verfahren nach einem der vorangehenden Ansprüche, wobei das angereicherte Erythropoietin nach der zweiten Anionenaustauschchromatographie eine Aktivität von ≥ 100.0001.E./mg aufweist.

18. Verfahren zur Herstellung einer Erythropoietin-Flüssigformulierung, umfassend die folgenden Schritte:
i) Anreichern von Erythropoietin, hergestellt durch Kultivieren von eukaryontischen Wirtszellen in einem Kulturmedium, in einem Proteingemisch, wobei die folgenden Schritte a)-c) in der angegebenen Reihenfolge durchgeführt werden:
a) eine erste Anionenaustauschchromatographie,
b) eine Affnitätschromatographie, eine hydrophobe Interaktionschromatographie und eine Hydroxyapatitchromatographie in der angegebenen Reihenfolge, und
c) eine zweite Anionenaustauschchromatographie,
und
(ii) Formulieren des in Schritt i) angereicherten Erythropoietins mit pharmazeutisch annehmbaren Hilfsstoffen, wie Puffer, Salze und Stabilisatoren, in einer Flüssigformulierung.

## Claims

1. Method for enrichment of erythropoietin, produced by cultivating eukaryotic host cells in a culture medium, in a protein mixture, wherein the following steps a) to c) are carried out in the given order:
a) a first anion exchange chromatography,
b) an affinity chromatography, a hydrophobic interaction chromatography and a hydroxyapatite chromatography in the order given, and
c) a second anion exchange chromatography.

2. Method according to claim 1, wherein the affinity chromatography is a dye affinity chromatography.

3. Method according to any of the preceding claims, wherein the second anion exchange chromatography comprises an acidic washing step.

4. Method according to any of the preceding claims, further comprising at least one filtration step.

5. Method according to claim 4, wherein a filtration is carried out after the final chromatographic purification step of step b).

6. Method according to claim 4 or 5, wherein a filtration is carried out after the second anion exchange chromatography.

7. Method according to any of the preceding claims, wherein the protein mixture is a host cell-free filtrate of the culture medium, which is subjected to diafiltration prior to the first anion exchange chromatography.

8. Method according to claim 7, wherein the host cell-free filtrate is subjected to ultrafiltration prior to the diafiltration.

9. Method according to any of the preceding claims, wherein the culture medium is protein-free and free of animal components.

10. Method according to any of the preceding claims, wherein the method does not comprise reversed-phase chromatography.

11. Method according to any of the preceding claims, wherein the method does not comprise gel filtration chromatography.

12. Method according to any of the preceding claims, wherein the method does not comprise protein precipitation.

13. Method according to any of the preceding claims, wherein the method does not comprise any further chromatographic purification steps.

14. Method according to any of the preceding claims, wherein the enriched erythropoietin has a host cell protein content of <100 ppm after the second anion exchange chromatography.

15. Method according to any of the preceding claims, wherein the enriched erythropoietin has a host cell DNA content of <100 pg/mg after the second anion exchange chromatography.

16. Method according to any of the preceding claims, wherein the enriched erythropoietin after the second anion exchange chromatography has a purity of>98%, determined by RP-HPLC.

17. Method according to any of the preceding claims, wherein the enriched erythropoietin after the second anion exchange chromatography has an activity of ≥100,000 I.U./mg.

18. Method for the production of an erythropoietin liquid formulation, comprising the following steps:
i) concentrating the erythropoietin produced by cultivation of eukaryotic host cells in a culture medium, in a protein mixture, wherein the following steps a) to c) are carried out in the given order:
a) a first anion exchange chromatography,
b) an affinity chromatography, a hydrophobic interaction chromatography and a hydroxyapatite chromatography in the order given, and
c) a second anion exchange chromatography,
and
ii) formulating the erythropoietin that is enriched in step i) with pharmaceutically acceptable adjuvants, such as buffers, salts and stabilising agents, in form of a liquid formulation.

## Revendications

1. Procédé pour l'enrichissement de l'érythropoïétine, produit par cultivation des cellules hôtes eucariotiques dans un milieu de culture, dans un mélange protéinique, dans lequel les étapes suivantes sont effectuées :
a) une première chromatographie à échange d'anions,
b) une chromatographie d'affinité, une chromatographie d'interaction hydrophobe et une chromatographie d'hydroxylapatite dans l'ordre donnée, et
c) une deuxième chromatographie à échange d'anions.

2. Procédé selon la revendication 1, dans lequel la chromatographie d'affinité est réalisée comme chromatographie d'affinité de colorant.

3. Procédé selon l'une des revendications précédentes, dans lequel la deuxième chromatographie à échange d'anions comprend une étape de lavage acide.

4. Procédé selon l'une des revendications précédentes comprenant en outre au moins une étape de filtration.

5. Procédé selon la revendication 4, dans lequel une filtration aura lieu après l'étape de purification chromatographique dernière de l'étape b).

6. Procédé selon la revendication 4 ou 5, dans lequel une filtration aura lieu après la deuxième chromatographie à échange d'anions.

7. Procédé selon l'une des revendications précédentes, dans lequel le mélange protéinique est un filtrat exempt de cellules hôtes du milieu de culture qui est soumis à une diafiltration avant la première chromatographie à échange d'anions .

8. Procédé selon la revendication 7, dans lequel le filtrat exempt de cellules hôtes est soumis à une ultrafiltration avant la diafiltration.

9. Procédé selon l'une des revendications précédentes, dans lequel le milieu de culture est exempt des protéines et de composants d'animals.

10. Procédé selon l'une des revendications précédentes, le procédé ne comprenant aucune chromatographie en phase reverse.

11. Procédé selon l'une des revendications précédentes, le procédé ne comprenant aucune filtration sur gel.

12. Procédé selon l'une des revendications précédentes, le procédé ne comprenant aucune précipitation de protéine.

13. Procédé selon l'une des revendications précédentes, le procédé en outre ne comprenant pas d'autres étapes de purification.

14. Procédé selon l'une des revendications précédentes, dans lequel l'érythropoïétine enrichi a une teneur en protéine de cellules hôtes de < 100 ppm après la deuxième chromatographie à échange d'anions.

15. Procédé selon l'une des revendications précédentes, dans lequel l'érythropoïétine enrichi a, après la deuxième chromatographie à échange d'anions, une teneur en DNA de cellules hôtes de < 100 pg/mg.

16. Procédé selon l'une des revendications précédentes, dans lequel l'érythropoïétine enrichi a, après la deuxième chromatographie à échange d'anions, une pureté de ≥ 98 %, déterminée au moyen de HPLC en RP.

17. Procédé selon l'une des revendications précédentes, dans lequel l'érythropoïétine enrichi a, après la deuxième chromatographie à échange d'anions, une activité de ≥ 100000 I.E. /mg.

18. Procédé pour la production d'une formulation liquide d'érythropoïétine, comprenant les étapes suivantes :
i) enrichissement de l'érythropoïétine, produit par cultivation des cellules hôtes eucariotiques dans un milieu de culture, dans un mélange protéinique en mettant en oeuvre les étapes a) - c) dans l'ordre donnée :
a) une première chromatographie à échange d'anions,
b) une chromatographie d'affinité, une chromatographie d'interaction hydrophobe et une chromatographie d'hydroxylapatite dans l'ordre donnée, et
c) une deuxième chromatographie à échange d'anions.
et
ii) formulation dans une formulation liquide de l'érythropoïétine enrichi dans l'étape i) avec des adjuvants pharmaceutiquement acceptables, comme les tampons, les sels et les stabilisateurs.
